# FASCICULE DE BREVET EUROPEEN

(11) **EP 3 008 016 B1**
(45) Date de publication et mention de la délivrance du brevet: **09.08.2017**
(21) Numéro de dépôt: 14736990.4
(22) Date de dépôt: 12.06.2014
(51) Int. Cl.: C02F 1/74, C02F 3/28

(54) **PROCEDE ET INSTALLATION DE DESULFURATION DU DIGESTAT ET DU BIOGAZ D'UN DIGESTEUR**
VERFAHREN UND ANLAGE ZUR ENTFERNUNG VON SCHWEFEL AUS DEM GÄRREST UND DEM BIOGAS EINES GÄRUNGSBEHÄLTERS
METHOD AND INSTALLATION FOR REMOVING SULPHUR FROM THE DIGESTATE AND THE BIOGAS OF A DIGESTER

(30) Priorité: 14.06.2013 FR 1355538
(43) Date de publication de la demande: 20.04.2016
(73) Titulaire: R+I Alliance, 92040 Paris La Defense (FR)
(72) Inventeur: LESTY, Yves, Las Condes (CL); CHAMY, Rolando, Valparaiso (CL)
(74) Mandataire: Cabinet Armengaud Aîné
(86) Numéro de dépôt international: PCT/IB2014/062171
(87) Numéro de publication internationale: WO 2014/199334

(56) Documents cités:
- WO-A1-2012/090139
- FR-A1- 2 956 657
- US-B1- 8 366 932

## Description

L'invention est relative à un procédé de désulfuration total ou partiel du digestat et du biogaz dans un digesteur d'effluents urbains et/ou agricoles et/ou industriels, en voie humide et/ou sèche, le digesteur étant constitué d'une enceinte fermée dans laquelle a lieu une digestion anaérobie d'une masse de produits à traiter, formant le digestat, avec un volume gazeux au-dessus du digestat d'où est extrait le biogaz, procédé selon lequel du digestat est prélevé en un point du digesteur, puis est réinjecté en un autre point.

Un procédé de ce genre est connu, notamment d'après le document WO 2012/090139.

Dans le secteur des eaux usées, le traitement des boues prend une importance croissante, non seulement en termes de réduction des déchets, mais aussi pour contribuer à la production d'énergie par des procédés de digestion anaérobie.

La digestion anaérobie des boues de station d'épuration produit des quantités significatives de biogaz, qui est un gaz riche en méthane pouvant être utilisé pour la production d'énergie ou de biocombustible.

Ce biogaz, toutefois, contient certains composés qui rendent difficile son utilisation selon l'usage final choisi. Parmi ces composés, on trouve le sulfure d'hydrogène H₂S. Ce composé est nuisible pour la santé humaine et a un effet corrosif sur les équipements. Il est présent selon des concentrations qui peuvent typiquement varier entre 0 et 10 000 ppm. Ce composé constitue la cible principale à éliminer du biogaz.

Divers procédés ont été développés pour éliminer le mieux possible le sulfure d'hydrogène. Parmi ces procédés, ceux du genre visé par l'invention permettent d'éviter l'accumulation de sulfure d'hydrogène dans le biogaz pendant le procédé de digestion anaérobie.

Selon le procédé de WO 2012/090139, une injection d'agent oxydant, gazeux ou liquide, est effectuée dans le digestat se déplaçant dans une boucle de recirculation. La dissolution complète de l'agent oxydant, notamment de l'air ou de l'oxygène, dans le digestat n'est pas toujours obtenue de manière satisfaisante de sorte qu'un courant diphasique de digestat et d'agent oxydant peut être induit, conduisant à une réaction dans la phase gazeuse avec dépôt de soufre sur les parois du digesteur.

L'invention a pour but, surtout, de proposer un procédé de désulfuration, du genre défini précédemment, qui permet d'obtenir, dans la plupart des cas, une dissolution complète de l'agent oxydant, en particulier de l'air ou de l'oxygène, dans le digestat, et qui permette de réduire la fréquence des opérations d'entretien du digesteur, notamment de nettoyage de ses parois. Il est souhaitable en outre que le procédé soit d'une exploitation simple et économique et puisse être mis en oeuvre aisément sur un digesteur existant.

Selon l'invention, un procédé de désulfuration du digestat et du biogaz, du genre défini précédemment, est caractérisé en ce que l'on fait effectuer au digestat prélevé :
- un premier trajet ascendant avec injection d'oxydant gazeux en partie basse pour aération, l'oxydant gazeux et le digestat circulant à co-courant,
- et un second trajet descendant, en condition anaérobie, avant retour au digesteur,
- l'oxydant gazeux en excès étant évacué dans la partie haute de l'un au moins des trajets.

De préférence, le digestat est mis en contact avec l'oxydant gazeux dans un dispositif séparé du digesteur, pour une dissolution efficace de l'oxydant gazeux.

Lorsque le digesteur comporte au moins une boucle de recirculation externe du digestat, le premier trajet ascendant et le second trajet descendant pour le digestat sont avantageusement prévus dans la boucle de recirculation.

De préférence, le premier trajet ascendant est effectué dans une chambre d'aération s'étendant essentiellement verticalement, tandis que le second trajet descendant est effectué dans une chambre de désaération s'étendant essentiellement verticalement.

Le passage de la fin du premier trajet ascendant au début du second trajet descendant peut être assuré par déversement du digestat par-dessus le bord supérieur d'une cloison.

L'oxydant gazeux peut être de l'air.

Avantageusement, l'oxydant gazeux est injecté en partie basse de la chambre d'aération, l'arrivée d'effluent à traiter ayant lieu également en partie basse de la chambre d'aération.

L'invention est également relative à une installation de production de biogaz comprenant un digesteur d'effluents urbains et/ou agricoles et/ou industriels, en voie humide et/ou sèche, le digesteur étant constitué d'une enceinte fermée dans laquelle a lieu une digestion anaérobie d'une masse de produits à traiter formant un digestat, avec un volume gazeux au-dessus du digestat d'où est extrait le biogaz, laquelle installation comporte sur le digesteur un point de prélèvement de digestat et un point de réinjection dans le digesteur, cette installation étant caractérisée en ce qu'elle comporte :
- un moyen définissant un premier trajet ascendant du digestat prélevé avec un moyen d'injection d'oxydant gazeux en partie basse du trajet, pour une aération du digestat ;
- un second moyen définissant un deuxième trajet descendant, en condition anaérobie, avant retour au digesteur ;
- et un moyen d'évacuation de l'oxydant gazeux en excès dans la partie haute de l'un au moins des trajets.

De préférence, le moyen définissant le premier trajet ascendant est constitué par une chambre d'aération s'étendant essentiellement verticalement, tandis que le second moyen définissant le deuxième trajet descendant est constitué par une chambre de désaération s'étendant essentiellement verticalement.

Avantageusement, la chambre d'aération et la chambre de désaération sont situées dans une même enceinte, notamment de forme prismatique, disposée verticalement et divisée intérieurement en deux espaces, séparés par une cloison, correspondant aux chambres d'aération et de désaération. De préférence, la hauteur de la cloison est inférieure à celle de l'enceinte et l'alimentation en effluent de la chambre de désaération est assurée par déversement, à partir de la chambre d'aération, par-dessus le bord supérieur de la cloison.

Un tube vertical peut plonger dans la chambre d'aération pour injection de l'oxydant gazeux en partie basse de cette chambre, le tube vertical étant alimenté en oxydant gazeux sous pression à partir d'un compresseur dont la sortie est reliée au tube, lequel peut comporter à son extrémité inférieure un diffuseur favorisant la dispersion de l'oxydant gazeux dans le digestat.

Lorsque l'installation comporte au moins une boucle de recirculation externe du digestat, la chambre d'aération et la chambre de désaération sont avantageusement prévues dans la boucle de recirculation.

Le moyen d'évacuation est avantageusement constitué par un évent dans la partie haute des chambres d'aération et désaération, et la teneur en méthane du biogaz est enrichie grâce à l'entraînement de dioxyde de carbone supérieur à l'entraînement de méthane, dans le gaz d'évent au-dessus des chambres d'aération et désaération.

La teneur en azote du biogaz est diminuée, grâce à l'entraînement de l'azote dans le gaz d'évent au-dessus des chambres d'aération et désaération.

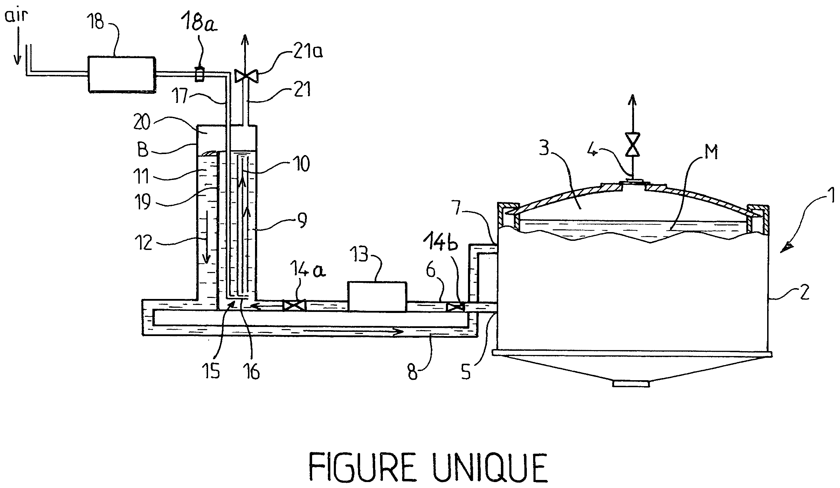

L'invention consiste, mises à part les dispositions exposées ci-dessus, en un certain nombre d'autres dispositions dont il sera plus explicitement question ci-après à propos d'un exemple de réalisation décrit avec référence au dessin annexé, mais qui n'est nullement limitatif.

La figure unique du dessin est un schéma d'une installation pour la mise en oeuvre du procédé conforme à l'invention.

Avant de décrire le procédé et l'installation avec référence à la figure du dessin, quelques rappels sont effectués à propos de la digestion anaérobie. Pendant une telle digestion, les sulfates contenus dans la boue sont réduits en sulfures par des bactéries réductrices de sulfates (SRB), et transférés ensuite à la phase gazeuse à travers l'équilibre liquide/gaz. Par une autre voie, la dégradation anaérobique de protéines génère aussi des sulfures.

Dans des conditions d'oxygénation limitée, et sans pour autant détourner la digestion de sa condition anaérobie, il est possible d'oxyder ces sulfures en soufre, à la place des sulfates, par un autre type de bactéries : les bactéries d'oxydation de sulfures (SOB).

L'invention consiste dans le développement d'un système ou procédé qui permet de dissoudre de petites quantités d'oxygène dans la boue ou digestat, favorisant la réaction de transformation directe de sulfures en soufre élémentaire dans la phase digestat (également appelée phase de boue), et en empêchant, en partie ou totalement, la libération de sulfure d'hydrogène H₂S dans le gaz. L'invention permet la dissolution de l'oxydant gazeux dans la boue, en évitant un courant diphasique de boue / digestat et d'oxydant gazeux, pouvant conduire à une réaction dans la phase gazeuse du digesteur.

Selon l'invention, l'injection d'oxydant gazeux est effectuée de telle sorte que le digestat ou boue est mis en contact avec l'oxydant gazeux, en général de l'air, dans un dispositif séparé du digesteur, notamment une chambre d'aération et une chambre de désaération comme expliqué ci-après.

En se reportant au dessin, on peut voir une installation comprenant un digesteur 1 constitué d'une enceinte 2 fermée dans laquelle a lieu une digestion anaérobie d'une masse M de produits à traiter formant un digestat avec un volume gazeux 3 au-dessus du digestat d'où est extrait le biogaz par une conduite 4.

Sur le dessin, les proportions ne sont pas respectées entre les dimensions du digesteur et celles des chambres d'aération et de désaération dont il sera question plus loin.

L'installation comporte sur le digesteur un point de prélèvement 5 de digestat, généralement en partie basse du digesteur auquel est raccordée une conduite 6. Le retour du digestat est effectué en un point de réinjection 7 généralement situé à un niveau supérieur à celui du point de prélèvement 5. Une conduite 8 de réinjection est raccordée au point 7. Une boucle de recirculation du digestat est ainsi formée par l'ensemble des conduites 6 et 8.

Dans cette boucle de recirculation est installée une chambre d'aération 9 s'étendant essentiellement verticalement et constituant un moyen pour définir un premier trajet ascendant 10 du digestat prélevé.

Une chambre de désaération 11 fait suite à la chambre 9 et constitue un moyen définissant un deuxième trajet descendant 12, en condition anaérobie, avant retour du digestat au digesteur par la conduite 8 reliée à la partie inférieure de la chambre 11.

Le digestat prélevé est mis en mouvement dans les conduites 6 et 8 par une pompe 13, notamment une pompe péristaltique, installée sur la conduite 6. Des vannes 14a et 14b disposées sur la conduite 6 permettent l'isolement de la pompe 13. Le débit du digestat prélevé peut être ajusté en agissant sur la vitesse de rotation de la pompe 13.

Un moyen 15 d'injection d'oxydant gazeux, de préférence de l'air, est prévu en partie basse du trajet ascendant 10, c'est-à-dire en partie basse de la chambre d'aération 9. Ce moyen d'injection 15 est avantageusement constitué par un diffuseur 16 placé à l'extrémité inférieure d'un tube vertical 17 plongeant dans le digestat de la chambre 9. L'alimentation en air comprimé est assurée par un compresseur 18 dont la sortie est reliée au tube 17. Un débitmètre 18a permet de contrôler le débit d'air introduit.

La chambre d'aération 9 et la chambre de désaération 11 sont de préférence situées dans une même enceinte B, notamment de forme prismatique, dont les génératrices sont disposées verticalement. Le volume intérieur de l'enceinte B est divisé en deux espaces séparés par une cloison 19 dont la hauteur est inférieure à celle de l'enceinte B de sorte qu'un espace 20, libre de digestat, se trouve en partie haute de l'enceinte B. L'alimentation en digestat de la chambre de désaération 11 est assurée par déversement du digestat, provenant de la chambre d'aération 9, par-dessus le bord supérieur de la cloison 19.

Un tube évent 21 débouche dans la partie supérieure de l'espace 20 pour permettre l'évacuation de l'air en excès qui ne s'est pas dissous dans le digestat lors du trajet ascendant 10. Cet évent 21 constitue un moyen d'évacuation de l'oxydant gazeux en excès dans la partie haute du trajet d'aération 10 qui correspond également à la partie haute du trajet de désaération 11 descendant. Le diffuseur 16 pour l'injection d'air/d'oxygène en partie inférieure de la chambre d'aération 9 favorise la dissolution de l'oxydant gazeux dans le digestat qui se produit lors de la circulation à co-courant suivant le trajet ascendant 10 du digestat et de l'air. Une soupape anti-retour 21a est installée sur le tube évent 21 pour permettre l'évacuation d'un gaz d'évent formé principalement d'air en excès et d'autres gaz, notamment dioxyde de carbone et méthane, entraînés. Plus de dioxyde de carbone CO₂ est entraîné dans le gaz d'évent que de méthane, le biogaz issu du digesteur sera donc enrichi en méthane.

Le digestat aéré est déversé en partie haute de la chambre d' aération 9, et tombe par gravité dans la chambre de désaération 11 constituant une zone anaérobie où il n'y a pas d'addition d'air/oxygène. Les bulles éventuelles restantes d'air/oxygène peuvent s'échapper du digestat par l'espace supérieur libre 20, ce qui permet d'éviter l'entrée de quantités d'air/oxygène trop importantes dans le digesteur 1. La conduite 8 assure le retour du digestat aéré vers le digesteur.

Le sulfure d'hydrogène qui était contenu dans le digestat prélevé au point 5 a été transformé, par l'injection d'oxydant, en soufre qui reste dans le digestat ou qui se dépose sur les parois de la chambre de désaération 11 ; par conséquent, le sulfure d'hydrogène est éliminé du biogaz, totalement ou partiellement, et les risques de dépôts sur les parois du digesteur 1 sont soit limités, soit annulés.

Bien que cela ne soit pas indispensable, il est avantageux de mettre en oeuvre la solution de l'invention dans des digesteurs qui utilisent une recirculation du digestat dans des buts de chauffage et/ou de mélange. Dans ce cas, le courant de recirculation du digestat est dirigé vers la chambre d'aération et la chambre de désaération. Il suffit d'inclure ces chambres dans la boucle de recirculation.

Un important avantage de l'installation proposée avec chambres d'aération et de désaération consiste en ce que la formation de soufre est évitée à l'intérieur du digesteur. Ceci est optimal du point de vue fonctionnement car une formation non contrôlée de soufre sur les parois et dans l'enceinte du digesteur peut conduite à des arrêts de fonctionnement non souhaités, à une diminution de la performance de digestion anaérobie et de la disponibilité de l'installation. Ces procédés de traitement ont une période de démarrage relativement longue jusqu'à une vingtaine de jours. De plus, une quantité moindre d'azote se retrouve dans le biogaz, une partie de l'azote étant éliminée par l'évent au-dessus des chambres d'aération et de désaération.

Des essais ont été effectuées dans une installation pilote comportant deux digesteurs jumeaux, équipés chacun d'une chambre d'aération 9 et d'une chambre de désaération 11. L'un de ces digesteurs fonctionnait sans injection d'air, tandis que l'autre digesteur fonctionnait avec injection d'air dans la chambre d'aération pour réaliser une micro-aération.

Les résultats comparatifs ont confirmé les avantages de l'invention concernant :
- la performance du dispositif proposé pour la micro-aération,
- la performance de la digestion anaérobie sous les effets de la micro-aération,
- les directives d'optimisation du procédé,
- une procédure d'augmentation du rendement.

Le procédé de l'invention engendre une micro-aération qui permet d'empêcher, partiellement ou totalement, la formation de sulfure d'hydrogène dans tout environnement de production de biogaz par une digestion anaérobie.

A titre d'exemple, il a été obtenu une réduction de 49,8% de sulfure d'hydrogène dans le biogaz, pour une micro-aération de 0,394 Nm³ d'air par m³ de digesteur et par jour ; la concentration en méthane dans le biogaz était 4 à 5% supérieure dans le digesteur micro-aéré, tandis que le dioxyde de carbone était proportionnellement plus bas ; la concentration en azote dans le biogaz de ce digesteur est restée toujours inférieure à 3%.

L'invention s'applique à tout environnement industriel qui comporte une production de biogaz, notamment stations de traitement d'eaux usées, décharges, digestion de produits agricoles pour la production d'énergie, et pour lesquelles le sulfure d'hydrogène H₂S, dans le biogaz, constitue un polluant cible qui doit être éliminé ou dont la formation doit être empêchée. Le cas échéant, d'autres avantages liés à la réduction du coût et au respect de l'environnement peuvent en résulter :
- si du chlorure ferrique FeCl3 est utilisé pour minimiser la production de sulfure d'hydrogène H₂S, cette consommation chimique coûteuse peut être réduite ou évitée ;
- des nettoyeurs chimiques et des matériaux d'absorption, par exemple du charbon actif, peuvent voir leur consommation réduite ;
- selon l'usage final et la concentration de sulfure d'hydrogène H₂S obtenue, tout investissement pour le traitement du biogaz pourrait être évité complètement ;
- pour un usage final tolérant, mais toutefois sensible au sulfure d'hydrogène, une augmentation de la durée de vie est assurée, par exemple pour un moteur à combustion interne ;
- l'émission d'oxydes de soufre, provenant de toute combustion d'un usage final, est réduite ou évitée.

Plus spécifiquement, le procédé de micro-aération selon l'invention fournit tous les bénéfices d'une micro-aération classique, en évitant un colmatage par le soufre à l'intérieur du digesteur, ce qui permet de réduire les problèmes de fonctionnement et des chutes de rendement de la digestion anaérobie. Il faut noter de plus, l'augmentation de la teneur en méthane du biogaz et la diminution de la teneur en dioxyde de carbone et en azote.

Tous ces résultats peuvent être obtenus avec un minimum d'investissement, puisqu'il suffit de prévoir une chambre d'aération et une chambre de désaération.

La mise en oeuvre est encore plus directe dans le cas des digesteurs qui utilisent une recirculation de digestat comme moyen de mélange, les chambres d'aération et de désaération étant alors installées dans la boucle.

## Revendications

1. Procédé de désulfuration du digestat et du biogaz dans un digesteur d'effluents urbains et/ou agricoles et/ou industriels, en voie humide et/ou sèche, le digesteur étant constitué d'une enceinte fermée dans laquelle a lieu une digestion anaérobie d'une masse de produits à traiter, formant le digestat, avec un volume gazeux au-dessus du digestat d'où est extrait le biogaz, procédé selon lequel du digestat est prélevé en un point du digesteur, puis est réinjecté en un autre point, **caractérisé en ce que** l'on fait effectuer au digestat prélevé :
- un premier trajet ascendant (10) avec injection d'oxydant gazeux en partie basse pour aération, l'oxydant gazeux et le digestat circulant à co-courant,
- et un second trajet descendant (12), en condition anaérobie, avant retour au digesteur,
- l'oxydant gazeux en excès étant évacué dans la partie haute (20) de l'un au moins des trajets.

2. Procédé selon la revendication 1, **caractérisé en ce que** le digestat est mis en contact avec l'oxydant gazeux dans un dispositif (B) séparé du digesteur, pour une dissolution efficace de l'oxydant gazeux.

3. Procédé selon la revendication 1 ou 2, pour un digesteur comportant au moins une boucle de recirculation externe du digestat, **caractérisé en ce que** le premier trajet ascendant et le second trajet descendant pour le digestat sont prévus dans la boucle de recirculation.

4. Procédé selon l'une quelconque des revendications précédentes, **caractérisé en ce que** le premier trajet ascendant (10) est effectué dans une chambre d'aération (9) s'étendant essentiellement verticalement, tandis que le second trajet descendant (12) est effectué dans une chambre de désaération (11) s'étendant essentiellement verticalement.

5. Procédé selon la revendication 4, **caractérisé en ce que** le passage de la fin du premier trajet ascendant (10) au début du second trajet descendant (12) est assuré par déversement du digestat par-dessus le bord supérieur d'une cloison.

6. Procédé selon l'une quelconque des revendications précédentes, **caractérisé en ce que** l'oxydant gazeux est de l'air.

7. Procédé selon la revendication 4, **caractérisé en ce que** l'oxydant gazeux est injecté en partie basse de la chambre d'aération (9), l'arrivée d'effluent à traiter ayant lieu également en partie basse de la chambre d'aération.

8. Installation de production de biogaz comprenant un digesteur (1) d'effluents urbains et/ou agricoles et/ou industriels, en voie humide et/ou sèche, le digesteur étant constitué d'une enceinte (2) fermée dans laquelle a lieu une digestion anaérobie d'une masse de produits à traiter formant un digestat, avec un volume gazeux au-dessus du digestat d'où est extrait le biogaz, laquelle installation comporte sur le digesteur un point de prélèvement (5) de digestat et un point de réinjection (7) dans le digesteur, cette installation étant **caractérisée en ce qu'**elle comporte :
- un moyen définissant un premier trajet ascendant (10) du digestat prélevé avec un moyen d'injection (15) d'oxydant gazeux en partie basse du trajet, pour une aération du digestat ;
- un second moyen définissant un deuxième trajet descendant (12), en condition anaérobie, avant retour au digesteur ;
- et un moyen d'évacuation (21) de l'oxydant gazeux en excès dans la partie haute de l'un au moins des trajets.

9. Installation selon la revendication 8, **caractérisée en ce que** le moyen définissant le premier trajet ascendant est constitué par une chambre d'aération (9) s'étendant essentiellement verticalement, tandis que le second moyen définissant le deuxième trajet descendant est constitué par une chambre de désaération (11) s'étendant essentiellement verticalement.

10. Installation selon la revendication 9, **caractérisée en ce que** la chambre d'aération (9) et la chambre de désaération (11) sont situées dans une même enceinte (B), notamment de forme prismatique, disposée verticalement et divisée intérieurement en deux espaces, séparés par une cloison (19), correspondant aux chambres d'aération et de désaération.

11. Installation selon la revendication 10, **caractérisée en ce que** la hauteur de la cloison (19) est inférieure à celle de l'enceinte (B) et l'alimentation en effluent de la chambre de désaération (11) est assurée par déversement, à partir de la chambre d'aération, par-dessus le bord supérieur de la cloison (19).

12. Installation selon la revendication 10 ou 11, **caractérisée en ce qu'**un tube vertical (17) plonge dans la chambre d'aération (9) pour injection de l'oxydant gazeux en partie basse de cette chambre, le tube vertical étant alimenté en oxydant gazeux sous pression à partir d'un compresseur (18) dont la sortie est reliée au tube.

13. Installation selon la revendication 12, **caractérisée en ce que** le tube (17) d'injection de l'oxydant gazeux comporte à son extrémité inférieure un diffuseur (16) favorisant la dispersion de l'oxydant gazeux dans le digestat.

14. Installation selon la revendication 9 ou 10, comportant au moins une boucle de recirculation externe du digestat, **caractérisée en ce que** la chambre d'aération et la chambre de désaération sont prévues dans la boucle de recirculation.

15. Installation selon la revendication 9, **caractérisée en ce que** le moyen d'évacuation (21) est constitué par un évent dans la partie haute des chambres d'aération et désaération, et la teneur en méthane du biogaz est enrichie grâce à l'entraînement de dioxyde de carbone supérieur à l'entraînement de méthane, dans le gaz d'évent au-dessus des chambres d'aération et désaération.

16. Installation selon la revendication 15, **caractérisée** en ce la teneur en azote du biogaz est diminuée, grâce à l'entraînement de l'azote dans le gaz d'évent au-dessus des chambres d'aération et désaération.

## Patentansprüche

1. Verfahren zur Entschwefelung des Gärrests und des Biogases eines Gärungsbehälters für kommunale und/oder landwirtschaftliche und/oder industrielle Abwässer, im Nass- und/oder Trockenverfahren, wobei der Gärungsbehälter aus einem geschlossenen Gehäuse gebildet ist, in welchem eine anaerobe Gärung einer aufzubereitenden Produktmasse stattfindet, welche den Gärrest bildet, mit einem über dem Gärrest befindlichen Gasvolumen, aus welchem das Biogas extrahiert wird, wobei bei dem Verfahren an einer Stelle des Gärungsbehälters Gärrest entnommen und anschließend an einer anderen Stelle wieder eingespritzt wird, **dadurch gekennzeichnet, dass** der entnommene Gärrest
- einen ersten, aufsteigenden Weg (10) durchläuft, wobei zur Belüftung gasförmiges Oxidationsmittel im unteren Teil eingespritzt wird, wobei das gasförmige Oxidationsmittel und der Gärrest im Gleichstrom umlaufen,
- und einen zweiten, absteigenden Weg (12) unter anaeroben Bedingungen vor der Rückkehr in den Gärungsbehälter durchläuft,
- wobei das überschüssige gasförmige Oxidationsmittel im oberen Teil (20) mindestens eines der Wege abgezogen wird.

2. Verfahren nach Anspruch 1, **dadurch gekennzeichnet, dass** der Gärrest in einer von dem Gärungsbehälter getrennten Vorrichtung (B) in Kontakt mit dem gasförmigen Oxidationsmittel gebracht wird, um eine effiziente Auflösung des gasförmigen Oxidationsmittels zu erreichen.

3. Verfahren nach Anspruch 1 oder 2, für einen Gärungsbehälter mit mindestens einem externen Gärrest-Rezirkulationskreislauf, **dadurch gekennzeichnet, dass** der erste, ansteigende Weg und der zweite, absteigende Weg für den Gärrest in dem Rezirkulationskreislauf vorgesehen sind.

4. Verfahren nach einem der vorhergehenden Ansprüche, **dadurch gekennzeichnet, dass** der erste, ansteigende Weg (10) in einer Belüftungskammer (9) verläuft, die sich im Wesentlichen vertikal erstreckt, während der zweite, absteigende Weg (12) in einer Entlüftungskammer (11) verläuft, die sich im Wesentlichen vertikal erstreckt.

5. Verfahren nach Anspruch 4, **dadurch gekennzeichnet, dass** der Übergang von dem Ende des ersten, ansteigenden Wegs (10) zum Anfang des zweiten, absteigenden Wegs (12) durch das Überlaufen des Gärrests über den oberen Rand einer Trennwand sichergestellt wird.

6. Verfahren nach einem der vorhergehenden Ansprüche, **dadurch gekennzeichnet, dass** das gasförmige Oxidationsmittel Luft ist.

7. Verfahren nach Anspruch 4, **dadurch gekennzeichnet, dass** das gasförmige Oxidationsmittel im unteren Teil der Belüftungskammer (9) eingespeist wird, wobei der Zufluss des aufzubereitenden Abwassers ebenfalls im unteren Teil der Belüftungskammer erfolgt.

8. Biogaserzeugungsanlage mit einem Gärungsbehälter (1) für kommunale und/oder landwirtschaftliche und/oder industrielle Abwässer, im Nass- und/oder Trockenverfahren, wobei der Gärungsbehälter aus einem geschlossenen Gehäuse (2) gebildet ist, in welchem eine anaerobe Gärung einer aufzubereitenden Produktmasse stattfindet, welche den Gärrest bildet, mit einem über dem Gärrest befindlichen Gasvolumen, aus welchem das Biogas extrahiert wird, wobei die Anlage an dem Gärungsbehälter eine Entnahmestelle (5) für Gärrest und eine Wiedereinspeisestelle (7) in den Gärungsbehälter aufweist, **dadurch gekennzeichnet, dass** die Anlage aufweist:
- eine Einrichtung, die einen ersten, aufsteigenden Weg (10) des entnommenen Gärrests bildet, mit einer Einrichtung (15) zum Einspeisen von gasförmigem Oxidationsmittel im unteren Teil des Weges zur Belüftung des Gärrests;
- eine zweite Einrichtung, die einen zweiten, absteigenden Weg (12) unter anaeroben Bedingungen vor der Rückkehr zum Gärungsbehälter bildet,
- und eine Einrichtung (21) zum Abziehen des überschüssigen gasförmigen Oxidationsmittels im oberen Teil mindestens eines der Wege.

9. Anlage nach Anspruch 8, **dadurch gekennzeichnet, dass** die den ersten, ansteigenden Weg bildende Einrichtung durch eine Belüftungskammer (9) gebildet ist, die sich im Wesentlichen vertikal erstreckt, während die den zweiten, absteigenden Weg bildende zweite Einrichtung durch eine Entlüftungskammer (11) gebildet ist, die sich im Wesentlichen vertikal erstreckt.

10. Anlage nach Anspruch 9, **dadurch gekennzeichnet, dass** die Belüftungskammer (9) und die Entlüftungskammer (11) in demselben, insbesondere prismenförmigen Gehäuse (B) angeordnet sind, das vertikal angeordnet ist und im Inneren in zwei, durch eine Trennwand (19) voneinander getrennte Räume unterteilt ist, die der Belüftungs- und der Entlüftungskammer entsprechen.

11. Anlage nach Anspruch 10, **dadurch gekennzeichnet, dass** die Höhe der Trennwand (19) geringer als die Höhe des Gehäuses (B) ist, und die Zufuhr von Abwasser in die Entlüftungskammer (11) ist durch das Überlaufen aus der Belüftungskammer über den oberen Rand der Trennwand (19) gewährleistet.

12. Anlage nach Anspruch 10 oder 11, **dadurch gekennzeichnet, dass** ein vertikales Rohr (17) in die Belüftungskammer (9) eingetaucht ist, um gasförmiges Oxidationsmittel im unteren Teil dieser Kammer einzuspeisen, wobei das vertikale Rohr durch einen Kompressor (18), dessen Auslass mit dem Rohr verbunden ist, mit druckbeaufschlagtem gasförmigem Oxidationsmittel gespeist wird.

13. Anlage nach Anspruch 12, **dadurch gekennzeichnet, dass** das Rohr (17) zum Einspeisen von gasförmigem Oxidationsmittel an seinem unteren Ende einen Diffusor (16) aufweist, welcher die Verteilung des gasförmigen Oxidationsmittels in dem Gärrest fördert.

14. Anlage nach Anspruch 9 oder 10, mit mindestens einem externen Gärrest-Rezirkulationskreislauf, **dadurch gekennzeichnet, dass** die Belüftungskammer und die Entlüftungskammer in dem Rezirkulationskreislauf vorgesehen sind.

15. Anlage nach Anspruch 9, **dadurch gekennzeichnet, dass** die Evakuierungseinrichtung (21) durch eine Lüftungsöffnung im oberen Teil der Belüftungs- und Entlüftungskammern gebildet ist, und der Methangehalt des Biogases erhöht ist, da in dem über der Belüftungs- und der Entlüftungskammer befindlichen Gas, welches durch die Lüftungsöffnung abgeführt wird, mehr Kohlendioxid als Methan mitgeführt wird.

16. Anlage nach Anspruch 15, **dadurch gekennzeichnet, dass** der Stickstoffgehalt des Biogases durch das Mitführen von Stickstoff in dem über der Belüftungs- und der Entlüftungskammer befindlichen Abgas verringert wird.

## Claims

1. A method for desulfurizing the digestate and the biogas in a digester for municipal and/or agricultural and/or industrial effluents, by wet and/or dry route, the digester being made up of a closed vessel in which a mass of products for treatment is anaerobically digested, forming the digestate, the biogas being extracted from a gas space above the digestate, in which method the digestate is withdrawn at one point in the digester and then reinjected at another point, **characterized in that** the digestate withdrawn is subjected to:
- an upward first path (10) with injection of gaseous oxidizing agent at the bottom part for aeration, the gaseous oxidizing agent and the digestate circulating cocurrently,
- and a downward second path (12), under anaerobic conditions, before return to the digester,
- the excess gaseous oxidizing agent being evacuated in the top part (20) of at least one of the paths.

2. The method as claimed in claim 1, **characterized in that** the digestate is contacted with the gaseous oxidizing agent in a separate device (B) of the digester, for effective dissolution of the gaseous oxidizing agent.

3. The method as claimed in claim 1 or 2, for a digester comprising at least one external digestate recirculation loop, **characterized in that** the upward first path and the downward second path for the digestate are provided in the recirculation loop.

4. The method as claimed in any of the preceding claims, **characterized in that** the upward first path (10) is effected in an aeration chamber (9) extending essentially vertically, while the downward second path (12) is effected in a deaeration chamber (11) extending essentially vertically.

5. The method as claimed in claim 4, **characterized in that** passage from the end of the upward first path (10) to the start of the downward second path (12) is ensured by pouring the digestate over the upper edge of a partition.

6. The method as claimed in any of the preceding claims, **characterized in that** the gaseous oxidizing agent is air.

7. The method as claimed in claim 4, **characterized in that** the gaseous oxidizing agent is injected at the bottom part of the aeration chamber (9), with effluent for treatment likewise entering at the bottom part of the aeration chamber.

8. A biogas production plant comprising a digester (1) for municipal and/or agricultural and/or industrial effluents, by wet and/or dry route, the digester being made up of a closed vessel (2) in which a mass of products for treatment is anaerobically digested, forming a digestate, the biogas being extracted from a gas space above the digestate, said plant including, on the digester, a point for withdrawal (5) of digestate and a point for reinjection (7) into the digester, this plant being **characterized in that** it comprises:
- a means defining an upward first path (10) of the digestate withdrawn, with a means (15) for injection of gaseous oxidizing agent at the bottom part of the path, for aeration of the digestate;
- a second means, defining a downward second path (12), under anaerobic conditions, before return to the digester;
- and a means (21) for evacuating excess gaseous oxidizing agent in the top part of at least one of the paths.

9. The plant as claimed in claim 8, **characterized in that** the means defining the upward first path consists of an aeration chamber (9) extending essentially vertically, while the second means, defining the downward second path, consists of a deaeration chamber (11) extending essentially vertically.

10. The plant as claimed in claim 9, **characterized in that** the aeration chamber (9) and the deaeration chamber (11) are situated in a single vessel (B), particularly a prism-shaped vessel, which is disposed vertically and is divided internally into two spaces, separated by a partition (19), corresponding to the aeration and deaeration chambers.

11. The plant as claimed in claim 10, **characterized in that** the height of the partition (19) is less than that of the vessel (B), and the deaeration chamber (11) is supplied with effluent by pouring, from the aeration chamber, over the upper edge of the partition (19).

12. The plant as claimed in claim 10 or 11, **characterized in that** a vertical tube (17) is submerged in the aeration chamber (9) for injection of the gaseous oxidizing agent at the bottom part of this chamber, the vertical tube being supplied with gaseous oxidizing agent under pressure from a compressor (18) whose outlet is connected to the tube.

13. The plant as claimed in claim 12, **characterized in that** the tube (17) for injection of the gaseous oxidizing agent comprises at its lower end a diffuser (16) which promotes the dispersion of the gaseous oxidizing agent in the digestate.

14. The plant as claimed in claim 9 or 10, comprising at least one external digestate recirculation loop, **characterized in that** the aeration chamber and the deaeration chamber are provided in the recirculation loop.

15. The plant as claimed in claim 9, **characterized in that** the means for evacuation (21) consists of a vent in the top part of the aeration and deaeration chambers, and the methane content of the biogas is enriched through the entrainment of carbon dioxide greater than the entrainment of methane, in the vent gas above the aeration and deaeration chambers.

16. The plant as claimed in claim 15, **characterized in that** the nitrogen content of the biogas is reduced through the entrainment of nitrogen in the vent gas above the aeration and deaeration chambers.
